(19) Europäisches Patentamt
European Patent Office
Office européen des brevets

(11) Veröffentlichungsnummer : **0 037 925**
**B1**

(12) # EUROPÄISCHE PATENTSCHRIFT

(45) Veröffentlichungstag der Patentschrift : **22.06.83**

(51) Int. Cl.$^3$ : **C 07 D237/14**, C 07 D237/16, C 07 D237/22, A 01 N 43/58

(21) Anmeldenummer : **81102167.4**

(22) Anmeldetag : **23.03.81**

(54) **Substituierte Pyridazone, Verfahren zu ihrer Herstellung, diese enthaltende Herbizide und ihre Anwendung als Herbizide.**

(30) Priorität : **05.04.80 DE 3013267**

(43) Veröffentlichungstag der Anmeldung : **21.10.81 Patentblatt 81/42**

(45) Bekanntmachung des Hinweises auf die Patenterteilung : **22.06.83 Patentblatt 83/25**

(84) Benannte Vertragsstaaten : **AT BE CH DE FR GB IT LI NL SE**

(56) Entgegenhaltungen : **DE A 2 526 643**

(73) Patentinhaber : **BASF Aktiengesellschaft**
**Carl-Bosch-Strasse 38**
**D-6700 Ludwigshafen (DE)**

(72) Erfinder : **Parg, Adolf, Dr.**
**Paray-le-Monial-Strasse 8**
**D-6702 Bad Duerkheim (DE)**
Erfinder : **Hamprecht, Gerhard, Dr.**
**Rote-Turm-Strasse 28**
**D-6940 Weinheim (DE)**
Erfinder : **Wuerzer, Bruno, Dr.**
**Ruedigerstrasse 13**
**D-6701 Otterstadt (DE)**

**EP 0 037 925 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Substituierte Pyridazone, Verfahren zu ihrer Herstellung, diese enthaltende Herbizide und ihre Anwendung als Herbizide

Die vorliegende Erfindung betrifft neue wertvolle substituierte Pyridazone, Verfahren zu ihrer Herstellung, ihre Anwendung als Herbizide und Herbizide, die diese Verbindungen als Wirkstoffe enthalten.

Es ist bekannt, daß 1-Phenyl-4,5-dimethoxy-pyridazon-(6) eine breite herbizide Wirkung hat und daher, ohne Kulturpflanzen zu schonen, als Totalherbizid verwendet werden kann (DE-PS 11 97 676). Der Wirkstoff besitzt pflanzenschädigende Eigenschaften sowohl bei Anwendung vor dem Auflaufen der Pflanzen als auch bei Behandlung der Blätter.

Wegen ähnlicher herbizider Eigenschaften wurde außerdem 1-m-Trifluormethylphenyl-4,5-dimethoxy-pyridazon-(6) bekannt (Belg. Patentschrift 728 164).

Es ist ferner bekannt, das 2-Chlor-4-Trifluormethylphenyl-3'-carboxy-4'-nitrophenylether-Natriumsalz als Herbizid zu verwenden (DE-OS 2 311 638).

Es ist ferner bekannt, Pyridazonderivate, die in 1-Stellung einen Phenylrest enthalten, der durch Fluoralkoxy substituiert ist, als Herbizide zu verwenden (DE-A-25 26 643). Diese bekannten Verbindungen unterscheiden sich von den erfindungsgemäßen Verbindungen in ihrer chemischen Struktur dadurch, daß die neuen Verbindungen an Stelle des Fluoralkoxi-Restes einen Phenoxyrest tragen. Die neuen Verbindungen werden daher durch die bekannten Verbindungen nicht nahegelegt.

Es wurde nun gefunden, daß substituierte Pyridazone der allgemeinen Formel

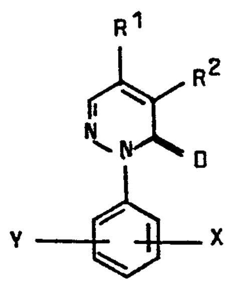

(I)

in der $R^2$ ein Halogenatom (Chlor, Brom) oder einen Alkoxyrest (Methoxy) mit 1 bis 3 Kohlenstoffatomen, $R^1$ eine Amino-, Alkylamino-, Dialkylamino-, Alkoxyamino- oder Alkylalkoxyaminogruppe mit 1 bis 3 Kohlenstoffatomen je Alkyl- bzw. Alkoxyrest, wobei die Alkylreste gleich oder verschieden sein können, ein Halogenatom (Chlor, Brom) einen Alkoxyrest mit 1 bis 3 Kohlenstoffatomen (Methoxy), einen Trimethyleniminorest oder eine durch $ClH_2CC(O)$- oder $CH_3COOCH_2(O)$ acylierte Aminogruppe, X einen substituierten Phenoxyrest der Formel

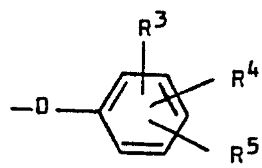

bedeutet, wobei $R^3$, $R^4$ und $R^5$ jeweils unabhängig voneinander Wasserstoff, Halogen (Chlor, Brom), Nitro, Cyano, Carboxyl, Alkyl (Methyl), Halogenalkyl (Trifluormethyl), Alkoxy (Methoxy), Halogenalkoxy, Alkylmercapto, Halogenalkylmercapto, Alkylsulfinyl oder Alkylsulfonyl mit jeweils 1 bis 4 Kohlenstoffatomen bedeuten und Y Wasserstoff, Halogen (Chlor), Halogenmethyl, Cyan oder Nitro bedeutet, eine gute herbizide Aktivität besitzen und dabei eine überraschende Verträglichkeit für Kulturpflanzen aufweisen.

$R^2$ in der Formel I kann beispielsweise Fluor, Chlor, Brom, Jod, Methoxy, Ethoxy oder Propoxy und $R^1$ kann beispielsweise Amino, Methylamino, Ethylamino, Dimethylamino, Diethylamino, Methylethylamino, Methoxyamino, Methylmethoxyamino, Chlor, Brom, Methoxy, Ethoxy, Propoxy, $ClCH_2C(O)NH$-, $CH_3COOCH_2C(O)NH$- oder einen Trimethyleniminorest bedeuten. X kann beispielsweise einen substituierten Phenoxyrest der Formel

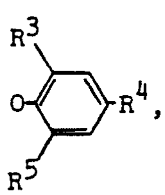, 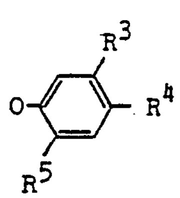 oder 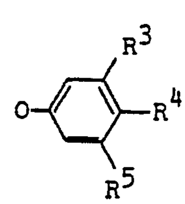

bedeuten, wobei $R^3$, $R^4$ und $R^5$ jeweils unabhängig voneinander beispielsweise Wasserstoff, Fluor,

Chlor, Brom, Jod, Nitro, Cyan, Carboxyl, Methyl, Äthyl, n-Propyl, i-Propyl, n-Butyl, tert.-Butyl, Trifluormethyl, Difluormethyl, Fluormethyl, Trichlormethyl, Dichlormethyl, Chlormethyl, Difluorchlormethyl, Methoxy, Äthoxy, n-Propyloxy, i-Propyloxy, tert.-Butyloxy, Trichlormethoxy, Trifluormethoxy, 1-Chloräthoxy, 2-Chloräthoxy, 1-Fluoräthoxy, 2-Fluoräthoxy, 2,2,2-Trichloräthoxy, 2,2,2-Trifluoräthoxy, 1,1,2,2-Tetrafluoräthoxy, 1,1,2,2,2-Pentafluoräthoxy, Methylmercapto, Äthylmercapto, Trichlormethylmercapto, Trifluormethylmercapto, Methylsulfinyl, Äthylsulfinyl, Methylsulfonyl oder Äthylsulfonyl bedeuten können.

Y kann beispielsweise Wasserstoff, Fluor, Chlor, Brom, Jod, Cyan oder Nitro bedeuten. Bevorzugte Verbindungen sind solche, bei denen $R^2$ beispielsweise Chlor, Brom oder Methoxy, $R^1$ beispielsweise Amino, Methylamino, Dimethylamino, Methylmethoxyamino oder Methoxy, X einen beispielsweise durch Halogen oder Halogenalkyl, insbesondere durch Chlor und Trifluormethyl substituierten Phenoxyrest in 3-oder 4-Stellung des Phenylrests und Y Wasserstoff oder Nitro in 6-Stellung des Phenylrests bedeuten.

Die Pyridazone der Formel I, wobei $R^1$ verschieden ist von Halogen, kann man herstellen durch Umsetzung eines Dihalogenpyridazons der Formel II

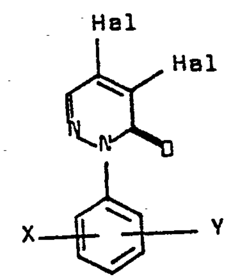

(II)

in der X und Y die obengenannten Bedeutungen haben und Hal Halogen, insbesondere Chlor oder Brom, bedeutet, mit der mindestens doppelt stöchiometrischen Menge eines Amins der Formel III

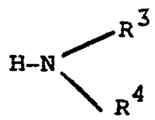

(III)

worin $R^3$ oder $R^4$ Wasserstoff, Alkyl mit 1 bis 3 Kohlenstoffatomen oder Alkoxy mit 1 bis 3 Kohlenstoffatomen bedeuten, oder mit ungefähr der stöchiometrischen Menge pro umzusetzendem Halogenatom — je nach dem, ob man eine oder beide Halogenatome umsetzen will — eines Alkoholats der Formel IV

$$MOR^5 \qquad (IV)$$

in der M ein Metallkation, insbesondere Natrium oder Kalium, und $R^5$ einen Alkylrest mit 1 bis 3 Kohlenstoffatomen (Methyl) bedeuten, in Gegenwart eines organischen Lösungsmittels bei einer Temperatur im Bereich zwischen 20 und 150 °C. Wobei man die Umsetzung drucklos oder unter Druck (1 bis 10 bar) kontinuierlich oder diskontinuierlich durchführt.

Verwendet man 1-[4'-(3'-Trifluormethyl-phenoxy)]-phenyl-4-5-dichlorpyridazon-(6)- und Natriummethylat als Ausgangsmaterialien, so kann der Reaktionsablauf durch folgendes Formelschema wiedergegeben werden :

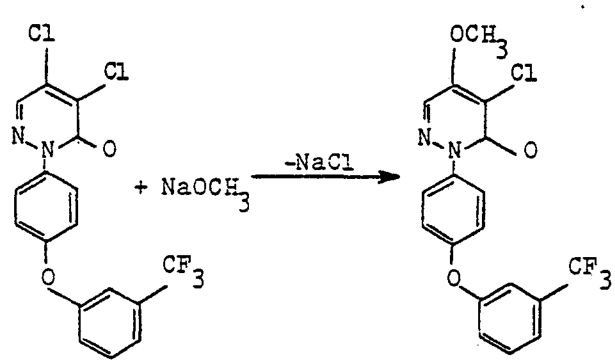


Zweckmäßigerweise wird das Dihalogenpyridazon II zunächst in einem organischen Lösungsmittel, beispielsweise in Ethanol, gelöst oder suspendiert und dann mit der entsprechenden Menge an Alkoholat oder Amin — dieses kann auch in Form einer wäßrigen oder alkoholischen Lösung eingesetzt werden — umgesetzt (DE-OS 25 26 643, DE-PS 12 10 241, US-PS 2 628 181, DE-OS 16 95 840). Die Reaktion kann

dabei unter normalem oder erhöhtem Druck, beispielsweise während 0,5 bis 12 Stunden bei einer Reaktionstemperatur von 20 bis 150 °C, vorzugsweise von 50 bis 120 °C, kontinuierlich oder diskontinuierlich durchgeführt werden. Die Aufarbeitung der Reaktionsmischung erfolgt nach allgemein üblichen Methoden. Fällt das Endprodukt fest an, so isoliert man es beispielsweise durch Absaugen des Niederschlages. Ist das endprodukt dagegen im Lösungsmittel gelöst, wird dieses unter vermindertem Druck abdestilliert, der Rückstand mit Wasser verrührt und abgesaugt. Zur Reinigung kann das Produkt beispielsweise umkristallisiert oder chromatographiert werden. Als Dihalogenpyridazone II können solche Verbindungen verwendet werden, die beispielsweise auf folgende Weise erhalten wurden :

Die Phenylhydrazine der Formel V

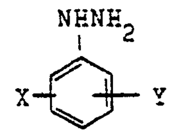

(V)

in der X und Y die obengenannten Bedeutungen besitzen, werden mit einer 3-Formyl-2,3-dihalogenacrylsäure der Formel VI

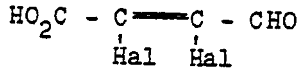

(VI)

in der Hal Halogen, insbesondere Chlor oder Brom bedeutet, vorzugsweise bei Raumtemperatur, in Gegenwart eines Lösungsmittels, z. B. einer mineralsauren wäßrigen Lösung oder einem wasserhaltigen oder wasserfreien, inerten organischen Lösungsmittel, wie Ethanol, das nach Beendigung der Reaktion abgedampft wird, zu dem entsprechenden Dihalogensäuresemicarbazon umgesetzt und dieses, beispielsweise ohne es zu isolieren, durch Kochen in Eisessig oder Essigsäureanhydrid oder durch Erwärmen in wäßriger Mineralsäure, z. B. Salzsäure, bei Temperaturen von 70 bis 100 °C oder durch Rühren in konzentrierter Mineralsäure, z. B. Schwefelsäure, bei Raumtemperatur (20 °C) zu der entsprechenden Verbindung der Formel II cyclisiert (DE-OS 16 95 840, DE-OS 25 26 643, DE-OS 15 45 595). Das Verfahren kann kontinuierlich oder diskontinuierlich erfolgen. Die Aufarbeitung der Reaktionsmischung erfolgt nach allgemein üblichen Methoden.

Die als Ausgangsstoffe benötigten Phenylhydrazine der Formel V können nach allgemein bekannten Methoden aus den entsprechenden Anilinen der Formel VII

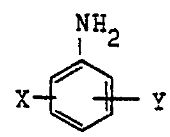

(VII)

in der X und Y die obengenannten Bedeutungen besitzen, in üblicher Weise durch Diazotierung und anschließende Reduktion des Diazoniumsalzes erhalten werden (Houben-Weyl, Methoden der organischen Chemie, Band 10/2, S. 180, Georg-Thieme-Verlag, Stuttgart, 1967). Die Umsetzung zum entsprechenden Pyrazon kann beispielsweise ohne Isolierung der Hydrazine erfolgen, jedoch erhält man reinere Endprodukte, wenn man die Phenylhydrazine als Hydrochloride isoliert.

Die Darstellung der Aniline der allgemeinen Formel VII kann für 4-Phenoxy-substituierte Derivate nach allgemein bekannten Methoden erfolgen (DE-OS 25 38 178, DE-OS 24 11 320), während die 3-Phenoxy-substituierten Derivate bekannt sind (Liebigs Ann. Chem. 740, 169-179 (1970)) oder nach dem bekannten Verfahren hergestellt werden können.

Die folgenden Beispiele erläutern die Herstellung der neuen Verbindungen der Formel I. Gewichtsteile verhalten sich zu Volumenteile wie kg zu l.

## Beispiel 1

25,3 Gew.-Teile 4-3'-Trifluormethylphenoxyanilin suspendiert in 230 Vol.-Teilen konz. Salzsäure werden mit 6,9 Gew.-Teilen $NaNO_2$ in 30 Vol.-Teilen $H_2O$ bei 0 °C diazotiert und anschließend mit 45 Gew.-Teilen $SnCl_2$ zu 4-3'-Trifluormethylphenoxyphenylhydrazinhydrochlorid reduziert. Das Hydrochlorid wird in 400 Vol.-Teilen 2N Salzsäure suspendiert, es werden 16,7 Gew.-Teile Mucochlorsäure zugesetzt und die Mischung 1 Stunde auf 90 °C gehalten. Nach dem Abkühlen trennt man die Salzsäure von der organischen Phase ab, versetzt die organische Phase mit 160 Vol.-Teilen Eisessig und rührt 10 Minuten unter Rückfluß. Man kühlt danach ab und fügt der Reaktionsmischung Wasser zu. Das entstandene 1-[4'-(3''-Trifluormethyl-phenoxy)]-phenyl-4,5-dichlor-pyridazon-6 wird abgesaugt und aus Methanol umkristallisiert (Nr. 1).

Ausbeute : 28,9 Gew.-Teile (72 % der Theorie)

Schmp. : 115-117 °C

Beispiel 2

28,7 Gew.-Teile 3-2'-Chlor-4'-trifluormethylphenoxy-anilin werden mit einer Lösung von 7,6 Gew.-Teilen $NaNO_2$ in 50 Vol.-Teilen konz. $H_2SO_4$ in 200 Vol.-Teilen Eisessig bei 10 bis 20 °C nitrosiert. Die Diazoniumsalzlösung wird direkt mit 45,5 Gew.-Teilen $SnCl_2$ gelöst in 31 Vol.-Teilen konz. Salzsäure zum entsprechenden hydrazin reduziert. Nach Zugabe von 16,7 Gew.-Teilen Mucochlorsäure rührt man die Reaktionslösung 10 Minuten bei Siedetemperatur, kühlt ab und fügt 1000 Vol.-Teile Wasser hinzu. Der ölige Rückstand wird in $CH_2Cl_2$ gelöst, mit $MgSO_4$ getrocknet, das Lösungsmittel abgedampft und der Rückstand durch Verreiben mit Methanol kristallisiert. Man erhält 32 Gew.-Teile (74 % der Theorie) 1-[3'-(2'-Chlor-4"-trifluormethyl-phenoxy)]-phenyl-4,5-dichlor-pyridazon-6 mit dem Schmelzpunkt 89 bis 92 °C (Nr. 2).

Beispiel 3

20 Gew.-Teile 1-[4'-(3"-Trifluormethyl-phenoxy)]-phenyl-4,5-dichlor-pyridazon-6 und 4 Gew.-Teile Natriummethylat werden in 80 Vol.-Teilen Methanol eine Stunde unter Rückfluß gekocht. Die Reaktionsmischung wird zur Trockne eingedampft, der Rückstand mit Wasser verrührt und abgesaugt. Nach dem Umkristallisieren aus Methanol erhält man 32 Gew.-Teile (80 % der Theorie) 1-[4'-(3"-Trifluormethyl-phenoxy)]-phenyl-4-methoxy-5-chlor-pyridazon-6 vom Schmelzpunkt 113 bis 114 °C (Nr. 3).

Beispiel 4

10 Gew.-Teile 1-[3'-(2"-Chlor-4"-trifluormethylphenoxy)]-phenyl-4,5-dichlor-pyridazon-6 und 6,3 Gew.-Teile Natriummethylat werden in 100 Vol.-Teilen absolutem Toluol eine Stunde zum Sieden erhitzt. Dann filtriert man heiß ab und dampft das Filtrat zur Trockene ein. Der ölige Rückstand wird mit Diisopropylether verrieben und 3,9 Gew.-Teile (35 % der Theorie) 1-[3'-(2"-Chlor-4"-trifluormethylphenoxy)]-phenyl-4,5-dimethoxy-pyridazon-6 mit dem Schmelzpunkt 72 bis 79 °C durch Absaugen erhalten (Nr. 4).

Beispiel 5

26 Gew.-Teile 1-[3'-(2"-Chlor-4"-trifluormethylphenoxy)]-phenyl-4,5-dichlorpyridazon-6 und 16 Teile Methylamin gelöst in 100 Vol.-Teilen Ethanol werden zusammen 1 Stunde zum Sieden erhitzt. Man filtriert heiß, engt das Filtrat zur Trockene ein und verrührt den Rückstand mit Wasser. Nach dem Absaugen und Umkristallisieren aus Methanol. Nach dem Absaugen und Umkristallisieren aus Methanol erhält man 15 Gew.-Teile (58 % der Theorie) 1-[3'-(2"-Chlor-4"-trifluormethylphenoxy)]-phenyl-4-methylamino-5-chlor-pyridazon-6 vom Schmelzpunkt 186 bis 188 °C (Nr. 5).

Beispiel 6

Eine Lösung von 20 Gew.-Teilen 1-[3'-(2"-Chlor-4"-trifluormethylphenoxy)]-phenyl-4,5-dichlorpyridazon-6 in 300 Vol.-Teilen Eisessig wird mit einer Mischung aus 5,6 Gew.-Teilen $HNO_3$ (Dichte 1,40) und 5,9 Gew.-Teilen konz. $H_2SO_4$ bei 0 °C bis 5 °C nitriert. Man rührt 2 Stunden bei 5 °C und fügt 1 000 Gew.-Teile Wasser zu. Der Niederschlag wird abgesaugt und der Rückstand aus Diisopropylether umkristallisiert. Man erhält 13,3 Gew.-Teile (60 % der Theorie) 1-[3'-(2"-Chlor-4"-trifluormethylphenoxy)-6'-nitro]-phenyl-4,5-dichlor-pyridazon-6 vom Schmelzpunkt 128 bis 130 °C (Nr. 6).

Beispiel 7

15 Gew.-Teile 1-[3'-(2"-Chlor-4"-trifluormethylphenoxy)-6'-nitro]-phenyl-4,5-dichlorpyridazon-6 und 200 Vol.-Teile konz. $NH_3$-Lösung werden bei 100 °C 12 Stunden unter Eigendruck im Autoklaven erhitzt. Man saugt den Rückstand ab und erhält nach Umkristallisieren aus Methanol 10 Gew.-Teile (72 % der Theorie) 1-[3'-(2"-Chlor-4"-trifluormethyl-phenoxy)-6'-nitro]-phenyl-4-amino-5-chlor-pyridazon-6 vom Schmelzpunkt 227 bis 230 °C (Nr. 7).

Entsprechend Beispiel 1 bis 7 werden die in der folgenden Tabelle angegebenen Verbindungen hergestellt :

(Siehe die Tabelle Seite 6)

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 8 | 2-Chlor-4-trifluor-methyl-phenoxy | 3 | H | $NH_2$ | Cl | 204-206 |
| 9 | " | 4 | " | " | " | |
| 10 | " | 3 | " | $OCH_3$ | " | 112-115 |
| 11 | " | 4 | " | " | " | |
| 12 | " | 3 | " | 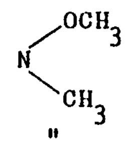 | " | 78- 82 |
| 13 | " | 4 | " | " | " | |
| 14 | " | 4 | " | $OCH_3$ | $OCH_3$ | |
| 15 | " | 4 | " | Cl | Cl | |
| 16 | " | 3 | " | 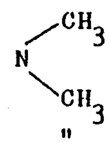 | " | |
| 17 | " | 4 | " | " | " | |
| 18 | 3-Chlor-4-trifluor-methyl-phenoxy | 3 | " | Cl | " | C=O 1670 $cm^{-1}$ |
| 19 | " | 4 | " | " | " | $n_D^{25}$ 1,5883 |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 20 | " | 3 | " | $OCH_3$ | " | 1,5987 |
| 21 | " | 4 | " | " | " | |
| 22 | " | 3 | " | " | $OCH_3$ | C=O 1650 $cm^{-1}$ |
| 23 | 3-Chlor-4-trifluor-methyl-phenoxy | 4 | H | $OCH_3$ | $OCH_3$ | 1,5749 |
| 24 | " | 3 | " | $NH_2$ | Cl | 154-158 |
| 25 | " | 4 | " | " | " | |
| 26 | " | 3 | " | 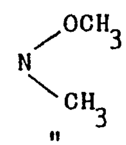 | " | |
| 27 | " | 4 | " | " | " | |
| 28 | " | 3 | " | 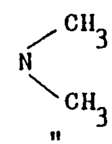 | " | |
| 29 | " | 4 | " | " | " | |
| 30 | 2,4-Dichlorphenoxy | 3 | " | Cl | Cl | |
| 31 | " | 4 | " | " | " | 122-124 |
| 32 | " | 3 | " | $OCH_3$ | " | |
| 33 | " | 4 | " | " | " | |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 34 | " | 3 | " | " | $OCH_3$ | |
| 35 | " | 4 | " | " | " | 126-131 |
| 36 | " | 3 | " | 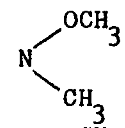 | Cl | |
| 37 | 2,4-Dichlorphenoxy | 4 | H | 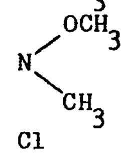 | Cl | |
| 38 | 2,6-Dichlor-4-trifluor-methyl-phenoxy | 3 | " | Cl | " | |
| 39 | " | 4 | " | " | " | |
| 40 | " | 3 | " | $OCH_3$ | " | |
| 41 | " | 4 | " | " | " | |
| 42 | " | 3 | " | " | $OCH_3$ | |
| 43 | " | 4 | " | " | " | |
| 44 | " | 3 | " | 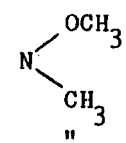 | Cl | |
| 45 | " | 4 | " | " | " | |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 46 | 2,4,6-Trichlorphenoxy | 3 | " | Cl | " | |
| 47 | " | 4 | " | " | " | |
| 48 | " | 3 | " | $OCH_3$ | " | |
| 49 | " | 4 | " | " | " | |
| 50 | " | 3 | " | " | $OCH_3$ | |
| 51 | " | 4 | " | " | " | |
| 52 | 2,4,6-Trichlorphenoxy | 3 | H | 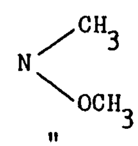 | Cl | |
| 53 | " | 4 | " | " | " | |
| 54 | 2,4-Difluorphenoxy | 3 | " | $OCH_3$ | $OCH_3$ | |
| 55 | " | 4 | " | " | " | |
| 56 | 2-Chlor-4-fluorphenoxy | 3 | " | " | " | |
| 57 | " | 4 | " | " | " | |
| 58 | 2,4-Dibromphenoxy | 3 | " | " | " | |
| 59 | " | 4 | " | " | " | |
| 60 | 2-Chlor-4-Nitro-phenoxy | 3 | " | " | " | |
| 61 | " | 4 | " | " | " | |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 62 | 2-Chlor-4-cyanophenoxy | 3 | " | " | " | |
| 63 | " | 4 | " | " | " | |
| 64 | 2-Chlor-4-methylmercapto-phenoxy | 3 | " | " | " | |
| 65 | " | 4 | " | " | " | |
| 66 | 2-Nitro-4-Trifluormethyl-phenoxy | 3 | " | " | " | |
| 67 | " | 4 | " | " | " | |
| 68 | 3-Trifluormethyl-4--chlor-phenoxy | 3 | " | " | " | |
| 69 | 3-Trifluormethyl-4--chlor-phenoxy | 4 | H | $OCH_3$ | $OCH_3$ | |
| 70 | 3-Trifluormethyl-4--nitro-phenoxy | 3 | " | " | " | |
| 71 | " | 4 | " | " | " | |
| 72 | 3,5-Dichlor-4-trifluormethyl-phenoxy | 3 | " | " | " | |
| 73 | " | 4 | " | " | " | |
| 74 | 3-Fluor-4-trifluormethyl-phenoxy | 3 | " | " | " | |
| 75 | " | 4 | " | " | " | |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 76 | 3-Cyano-4-trifluormethyl-phenoxy | 3 | " | " | " | |
| 77 | " | 4 | " | " | " | |
| 78 | 2,5-Dichlor-4-trifluormethyl-phenoxy | 3 | " | " | " | |
| 79 | " | 4 | " | " | " | |
| 80 | 2-Chlor-4-trifluormethoxy-phenoxy | 3 | " | " | " | |
| 81 | " | 4 | " | " | " | |
| 82 | 2,6-Dichlor-4-trifluormethoxy-phenoxy | 3 | " | " | " | |
| 83 | " | 4 | " | " | " | |
| 84 | 2-Chlor-4-difluormethyl-phenoxy | 3 | H | $OCH_3$ | $OCH_3$ | |
| 85 | " | 4 | " | " | " | |
| 86 | 2-Methyl-4-chlorphenoxy | 3 | " | " | " | |
| 87 | " | 4 | " | " | " | |
| 88 | 3-Trifluormethylphenoxy | 3 | " | " | " | |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 89 | " | 4 | " | " | " | 84-85 |
| 90 | " | 3 | " | $NHCH_3$ | Cl | |
| 91 | " | 4 | " | " | " | 131-134 |
| 92 | " | 3 | " | $NH_2$ | " | |
| 93 | " | 4 | " | " | " | 175-177 |
| 94 | 4-Chlorphenoxy | 4 | $3-CF_3$ | Cl | Cl | 129-131 |
| 95 | " | 4 | " | $OCH_3$ | " | 168-170 |
| 96 | " | 4 | " | " | $OCH_3$ | 98-100 |
| 97 | " | 4 | " | $NHCH_3$ | Cl | 156-158 |
| 98 | " | 4 | " | $NH_2$ | " | 199-201 |
| 99 | 2-Chlor-4-trifluor-methyl-phenoxy | 3 | $6-NO_2$ | $NH\underset{\overset{\|}{O}}{C}CH_2Cl$ | Cl | |
| 100 | " | 3 | " | $OCH_3$ | " | 179-181 |
| 101 | 2-Chlor-4-trifluor-methyl-phenoxy | 3 | $6-NO_2$ | $OCH_3$ | $OCH_3$ | 128-131 |
| 102 | " | 3 | " | $NHCH_3$ | Cl | 164-167 |
| 103 | " | 3 | " | 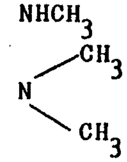 | Cl | 155-159 |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 104 | " | 3 | " | 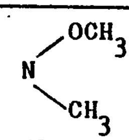 | Cl | 167-171 |
| 105 | " | 3 | 6-Cl | Cl | " | |
| 106 | " | 3 | " | $OCH_3$ | " | |
| 107 | " | 3 | " | $OCH_3$ | $OCH_3$ | |
| 108 | 2,6-Dichlor-4-tri-fluormethyl-phenoxy | 3 | $6-NO_2$ | " | " | |
| 109 | 2,4-Dichlorphenoxy | 3 | " | " | " | |
| 110 | 2,4,6-Trichlorphenoxy | 3 | " | " | " | |
| 111 | 3-Chlor-4-trifluor-methyl-phenoxy | 3 | H | $NHCH_3$ | H | C=O = 1620 $cm^{-1}$ |
| 112 | 2,5-Dichlorphenoxy | 4 | " | Cl | Cl | 81- 84 |
| 113 | " | 4 | " | $OCH_3$ | $OCH_3$ | 98-103 |
| 114 | 3-Bromphenoxy | 4 | " | Cl | Cl | 110-113 |
| 115 | " | 4 | " | $OCH_3$ | $OCH_3$ | 96- 99 |
| 116 | 2-Brom-4-chlorphenoxy | 4 | " | Cl | Cl | 128-131 |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 117 | 2-Brom-4-chlor-phenoxy | 4 | H | $OCH_3$ | $OCH_3$ | 111-115 |
| 118 | 2-Brom-4-fluor-phenoxy | 4 | " | Cl | Cl | 91- 93 |
| 119 | " | 4 | " | $OCH_3$ | $OCH_3$ | 100-105 |
| 120 | 3,4-Dichlorphenoxy | 4 | " | Cl | Cl | 137-142 |
| 121 | " | 4 | " | $OCH_3$ | $OCH_3$ | 101-103 |
| 122 | 3-Trifluormethoxyphenoxy | 4 | " | Cl | Cl | 84- 89 |
| 123 | " | 4 | " | $OCH_3$ | $OCH_3$ | 58- 63 |
| 124 | 3-Tert.butylphenoxy | 4 | " | Cl | Cl | 76- 77 |
| 125 | " | 4 | " | $OCH_3$ | $OCH_3$ | 1,5768 |
| 126 | 3-Methoxyphenoxy | 4 | " | Cl | Cl | 97-101 |
| 127 | " | 4 | " | $OCH_3$ | $OCH_3$ | 71- 76 |
| 128 | 4-Chlor-3-methylphenoxy | 4 | " | Cl | Cl | 160-163 |
| 129 | " | 4 | " | $OCH_3$ | $OCH_3$ | 83-86 |
| 130 | 3-Methylphenoxy | 4 | " | Cl | Cl | 130-132 |
| 131 | " | 4 | " | $OCH_3$ | $OCH_3$ | 79- 81 |

| Beispiel Nr. | X | Stellung X | Y | $R^1$ | $R^2$ | Fp [°C] $n_D^{25}$ Wellenlänge einer Bande im IR-Spektrum |
|---|---|---|---|---|---|---|
| 132 | 2-Chlor-5-methylphenoxy | 4 | " | Cl | Cl | 88- 92 |
| 133 | " | 4 | " | $OCH_3$ | $OCH_3$ | 109-112 |
| 134 | 2-Chlor-4-bromphenoxy | 4 | " | Cl | Cl | 130-132 |
| 135 | " | 4 | " | $OCH_3$ | $OCH_3$ | 138-140 |
| 136 | 3-Fluorphenoxy | 4 | " | Cl | Cl | 130-133 |
| 137 | " | 4 | " | $OCH_3$ | $OCH_3$ | 84- 88 |
| 138 | 3-Trifluormethylphenoxy | 4 | 3-Cl | Cl | Cl | 105-109 |
| 139 | " | 4 | " | $OCH_3$ | $OCH_3$ | 82- 89 |
| 140 | " | 4 | $3-CF_3$ | Cl | Cl | 84- 89 |
| 141 | " | 4 | " | $OCH_3$ | $OCH_3$ | 75- 82 |
| 142 | " | 4 | " | $NHCH_3$ | Cl | C=O 1620 $cm^{-1}$ |
| 143 | 2-Chlor-4-trifluor-methylphenoxy | 3 | $6-NO_2$ | $NH-C_3H_7(i)$ | Cl | 170-176 |
| 144 | " | 3 | " | $N(C_2H_5)_2$ | Cl | 142-143 |

Der Einfluß von Vertretern der neuen phenoxysubstituierten Pyridazone auf das Wachstum von erwünschten und unerwünschten Pflanzen wird anhand von Gewächshausversuchen vorgeführt:

Als Kulturgefäße dienten Plastikblumentöpfe mit 300 $cm^3$ Inhalt und lehmiger Sand mit etwa 1,5 % Humus als Substrat. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 3,0 kg Wirkstoff/ha. Nach dem Aufbringen der Mittel wurden die Gefäße leicht beregnet, um Keimung und Wachstum in Ganz zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 10 cm an und behandelte sie danach. Zur Nachauflaufbehandlung wurden entweder direkt gesäte und in den gleichen Gefäßen aufgewachsene Pflanzen ausgewählt, oder sie wurden erst als Keimpflanzen getrennt angezogen und einige Tage vor der Behandlung in die Versuchsgefäße verpflanzt. Die Aufwandmengen für die Nachauflaufbehandlung waren 0,25 kg/ha und 0,5 kg/ha Wirkstoff. Als Vergleichsmittel wurden die bekannten Wirkstoffe 1-Phenyl-4,5-dimethoxy-pyridazon-(6) (A), 1-m-Trifluor-methylphenyl-4,5-dimethoxypyridazon-(6) (B) und 2-Chlor-4-trifluormethylphenyl-3′-carboxy-4′-nitrophenylether-Natriumsalz (C) in den gleichen Aufwandmengen gewählt. Eine Abdeckung unterblieb bei der Nachauflaufbehandlung. Die Aufstellung der Versuche erfolgte im Gewächshaus, wobei für wärmeliebende Arten wärmere Bereiche (20 bis 30 °C) und für solche gemäßigter Klimate 15 bis 25 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die Ergebnisse zeigen, daß die neuen Verbindungen bei der vorzugsweise durchgeführten Nachauflaufbehandlung insgesamt eine ähnliche, gegen einige unerwünschte Arten sogar eine bessere herbizide Aktivität haben als der bekannte Vergleichswirkstoff. Besonders hervorzuheben ist jedoch die wesentlich günstigere Verträglichkeit der neuen Verbindungen für Kulturpflanzen, vorzugsweise aus der Familie der Gramieen. Bei Vorauflaufanwendung wird ebenfalls eine herbizide Wirkung beobachtet.

Sind gewisse Kulturpflanzen gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post directed, lay-by). In Anbetracht der guten Verträglichkeit und der Vielseitigkeit der Applikationsmethoden können die erfindungsgemäßen Herbizide noch in einer weiteren großen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

In Betracht kommen beispielsweise die folgenden Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Aventa sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. naprobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor-Färbedistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea cane phora, Coffea liberica) | Kaffee | coffee plants |

(Fortsetzung)

| Botanischer Name | Deutscher Name | Englischer Name |
| --- | --- | --- |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartofeln | sweet potato |
| Juglans regia | Walnußbaum | walnut trees |
| Lactuca sativa | Kopfsalat | lettuce |
| Lens culinaris | Linse | lentils |
| Linum usitatissimum | Faserlein | flax |
| Lycopersiocon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- u. Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beens, dry beans |
| Pennisetum glaucum | Perl- oder Rohrkolbenhirse | |
| Petroselinim crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Prunus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potato |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |

(Fortsetzung)

| Botanischer Name | Deutscher Name | Englischer Name |
|---|---|---|
| Vaccinium carymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | India corn, sweet corn, maize |

Zur Verbreiterung des Wirkungsspektrums und zur Erzielung synergistischer Effekte können die neuen phenoxysubstituierten Pyridazone sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Die Anwendung erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken ; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen :

Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylāthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, äthoxyliertes Isooctylphenol-, Octylphenol-, Nonylphenol, Alkylphenolpolyglykoläther. Tributylphenylpolyglykolether, Alkylarylpolyätheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiel a

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel b

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel c

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel d

20 Gewichtsteile der Verbindung 4 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel e

20 Gewichtsteile des Wirkstoffs 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel f

3 Gewichtsteile der Verbindung 2 werden mit 97 Gewichtsteilen feinteiligem Kaolin innig vermischt. Man erhält auf diese Weise ein Stäubemittel, das 3 Gewichtsprozent des Wirkstoffs enthält.

Beispiel g

30 Gewichtsprozent der Verbindung 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel h

40 Gewichtsteile des Wirkstoffs 4 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel i

20 Teile des Wirkstoffs 1 werden mit 12 Teilen Calciumsalz der Dodecylbenzolsulfonsäure, 8 Teile Fettalkoholpolyglykoläther, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Bei der Prüfung auf selektive herbizide Wirkung bei Nachauflaufanwendung im Gewächshaus bei

Aufwandmengen von 0,25 kg Wirkstoff/ha zeigt die neue Verbindung Nr. 4 eine bessere selektive herbizide Wirkung als der bekannte Wirkstoff A.

Bei der selektiven Unkrautbekämpfung bei Nachauflaufanwendung im Gewächshaus bei Aufwandmengen von 0,5 kg Wirkstoff/ha zeigt der neue Wirkstoff 88 eine bessere selektive herbizide Wirkung als der bekannte Wirkstoff B.

Bei der Prüfung der herbiziden Wirkung bei Vorauflaufanwendung im Gewächshaus bei Aufwandmengen von 3 kg Wirkstoff je ha zeigen die neuen Wirkstoffe 4 und 88 eine sehr gute herbizide Wirkung.

Bei der Prüfung der herbiziden Wirkung bei Nachauflaufanwendung im Gewächshaus bei Aufwandmengen von 0,25 kg Wirkstoff/ha zeigt der neue Wirkstoff 88 eine bessere selektive herbizide Wirkung als der bekannte Wirkstoff B.

Bei der Prüfung der herbiziden Wirkung bei Nachauflaufanwendung im Gewächshaus bei Aufwandmengen von 0,5 und 0,25 kg Wirkstoff je ha zeigten die neuen Wirkstoffe 7 und 102 eine bessere selektive herbizide Wirkung als der bekannte Wirkstoff C.

Bei der Prüfung der herbiziden Wirkung bei Nachauflaufanwendung im Gewächshaus bei Aufwandmengen von 0,5 kg Wirkstoff je ha zeigten die Wirkstoffe 87 und 126 eine gute herbizide Wirkung.

Bei der Prüfung der herbiziden Wirkung bei Nachauflaufanwendung im Gewächshaus bei Aufwandmengen von 0,25 kg Wirkstoff je ha zeigten die Wirkstoffe 22 und 101 eine gute herbizide Wirkung.

**Ansprüche** (für die Vertragsstaaten BE, CH, DE, FR, GB, IT, LI, NL, SE)

1. Substituierte Pyridazone der allgemeinen Formel

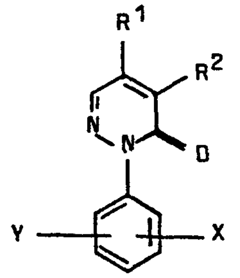

(I)

in der $R^2$ ein Halogenatom oder einen Alkoxyrest mit 1 bis 3 Kohlenstoffatomen, $R^1$ eine Amino-, Alkylamino-, Dialkylamino-, Alkoxyamino- oder Alkylalkoxyaminogruppe mit 1 bis 3 Kohlenstoffatomen je Alkyl- bzw. Alkoxyrest, wobei die Alkylreste gleich oder verschieden sein können, ein Halogenatom, einen Alkoxyrest mit 1 bis 3 Kohlenstoffatomen, einen Trimethyleniminorest oder eine durch $ClH_2CC(O)$- oder $CH_3COOCH_2C(O)$-acylierte Aminogruppe, X einen substituierten Phenoxyrest der Formel

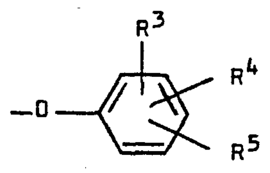

bedeutet, wobei $R^3$, $R^4$ und $R^5$ jeweils unabhängig voneinander Wasserstoff, Halogen, Nitro, Cyano, Carboxyl, Alkyl, Halogenalkyl, Alkoxy, Halogenalkoxy, Alkylmercapto, Halogenalkylmercapto, Alkylsulfinyl oder Alkylsulfonyl mit jeweils 1 bis 4 Kohlenstoffatomen bedeuten und Y Wasserstoff, Cyan, Halogenmethyl, Halogen oder Nitro bedeutet.

2. Herbizid, enthaltend ein Pyridazon gemäß Anspruch 1.

3. Herbizid, enthaltend einen festen oder flüssigen Trägerstoff und ein Pyridazon gemäß Anspruch 1.

4. Verfahren zur Herstellung eines Herbizids, dadurch gekennzeichnet, daß man einen festen oder flüssigen Trägerstoff vermischt mit einem Pyridazon gemäß Anspruch 1.

5. Verfahren zur Bekämpfung unerwünschten Pflanzenwuchses, dadurch gekennzeichnet, daß man die Pflanzen oder den Boden behandelt mit einem Pyridazon gemäß Anspruch 1.

6. Verfahren zur Herstellung eines Pyridazons gemäß Anspruch 1, dadurch gekennzeichnet, daß man ein Dihalogenpyridazon der Formel II

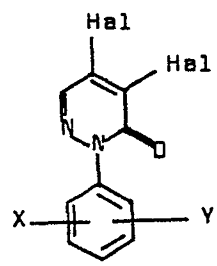

(II)

in der X und Y die oben genannten Bedeutungen haben und Hal Halogen bedeutet, mit der mindestens doppelt stöchiometrischen Menge eines Amins der Formel III

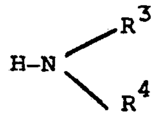 (III)

worin $R^3$ oder $R^4$ Wasserstoff, Alkyl mit 1 bis 3 Kohlenstoffatomen oder Alkoxy mit 1 bis 3 Kohlenstoffatomen bedeuten und $R^3$ und $R_4$ im Einzelfall gleich oder verschieden sind, oder mit ungefähr der stöchiometrischen Menge pro umzusetzendem Halogenatom eines Alkoholats der Formel IV

$$MOR^5 \qquad (IV)$$

in der M ein Metallkation, insbesondere Natrium oder Kalium, und $R^5$ einen Alkylrest mit 1 bis 3 Kohlenstoffatomen bedeutet, in Gegenwart eines organischen Lösungsmittels bei einer Temperatur im Bereich zwischen 20 und 150 °C, unter Druck oder drucklos, kontinuierlich oder diskontinuierlich umsetzt.

7. Pyridazon gemäß Anspruch 1, dadurch gekennzeichnet, daß $R^1$ Chlor oder Methoxy, $R^2$ Chlor oder Methoxy, $R^3$ Chlor oder Wasserstoff, $R^4$ Trifluormethyl, $R^5$ Wasserstoff und Y Nitro oder Wasserstoff bedeutet.

8. Herbizid, enthaltend ein Pyridazon gemäß Anspruch 1, dadurch gekennzeichnet, daß $R^1$ Chlor oder Methoxy, $R^2$ Chlor oder Methoxy, $R^3$ Chlor oder Wasserstoff, $R^4$ Trifluormethyl, $R^5$ Wasserstoff und Y Nitro oder Wasserstoff bedeutet.

**Ansprüche** (für den Vertragsstaat AT)

1. Herbizid, enthaltend ein Pyridazon der Formel

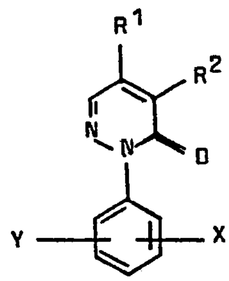 (I)

in der $R^2$ ein Halogenatom oder einen Alkoxyrest mit 1 bis 3 Kohlenstoffatomen, $R^1$ eine Amino-, Alkylamino-, Dialkylamino-, Alkoxyamino- oder Alkylalkoxyaminogruppe mit 1 bis 3 Kohlenstoffatomen je Alkyl-bzw.
Alkoxyrest, wobei die Alkylreste gleich oder verschieden sein können, ein Halogenatom, einen Alkoxyrest mit 1 bis 3 Kohlenstoffatomen, einen Trimethyleniminorest oder eine durch $ClH_2CC(O)$- oder $CH_3COOCH_2C(O)$-acylierte Aminogruppe, X einen substituierten Phenoxyrest der Formel

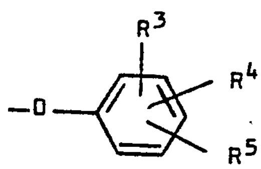

bedeutet, wobei $R^3$, $R^4$ und $R^5$ jeweils unabhängig voneinander Wasserstoff, Halogen, Nitro, Cyano, Carboxyl, Alkyl, Halogenalkyl, Alkoxy, Halogenalkoxy, Alkylmercapto, Halogenalkylmercapto, Alkylsulfinyl oder Alkylsulfonyl mit jeweils 1 bis 4 Kohlenstoffatomen bedeuten und Y Wasserstoff, Cyan, Halogenmethyl, Halogen oder Nitro bedeutet.

2. Herbizid, enthaltend einen festen oder flüssigen Trägerstoff und ein Pyridazon gemäß Anspruch 1.

3. Verfahren zur Herstellung eines Herbizids, dadurch gekennzeichnet, daß man einen festen oder flüssigen Trägerstoff vermischt mit einem Pyridazon gemäß Anspruch 1.

4. Verfahren zur Bekämpfung unerwünschten Pflanzenwuchses, dadurch gekennzeichnet, daß man die Planzen oder den Boden behandelt mit einem Pyridazon gemäß Anspruch 1.

5. Verfahren zur Herstellung eines Pyridazons gemäß Anspruch 1, dadurch gekennzeichnet, daß man ein Dihalogenpyridazon der Formel II

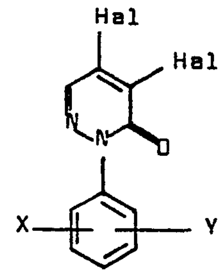

(II)

in der X und Y die oben genannten Bedeutungen haben und Hal Halogen bedeutet, mit der mindestens doppelt stöchiometrischen Menge eines Amins der Formel III

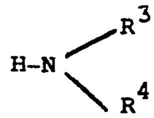

(III)

worin $R^3$ oder $R^4$ Wasserstoff, Alkyl mit 1 bis 3 Kohlenstoffatomen oder Alkoxy mit 1 bis 3 Kohlenstoffatomen bedeuten und $R^3$ und $R^4$ im Einzelfall gleich oder verschieden sind, oder mit ungefähr der stöchiometrischen Menge pro umzusetzendem Halogenatom eines Alkoholats der Formel IV

$$MOR^5 \qquad (IV)$$

in der M ein Metallkation, insbesondere Natrium oder Kalium, und $R^5$ einen Alkylrest mit 1 bis 3 Kohlenstoffatomen bedeutet, in Gegenwart eines organischen Lösungsmittels bei einer Temperatur im Bereich zwischen 20 und 150 °C, unter Druck oder drucklos, kontinuierlich oder diskontinuierlich umsetzt.

6. Herbizid, gemäß Anspruch 1, dadurch gekennzeichnet, daß $R^1$ Chlor oder Methoxy, $R^2$ Chlor oder Methoxy, $R^3$ Chlor oder Wasserstoff, $R^4$ Trifluormethyl, $R^5$ Wasserstoff und Y Nitro oder Wasserstoff bedeutet.

**Claims** (for the Contracting states : BE, CH, DE, FR, GB, IT, LI, NL, SE)

1. A substituted pyridazone of the general formula

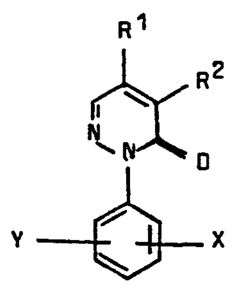

(I)

where $R^2$ is halogen or alkoxy of 1 to 3 carbon atoms, $R^1$ is amino, alkylamino, dialkylamino, alkoxyamino or alkylalkoxyamino, where alkyl and alkoxy are each of 1 to 3 carbon atoms and the alkyl radicals may be identical or different, halogen, alkoxy of 1 to 3 carbon atoms, trimethyleneimino or amino acylated by $ClH_2CC(O)$- or $CH_3COOCH_2C(O)$-, is substituted phenoxy of the formula

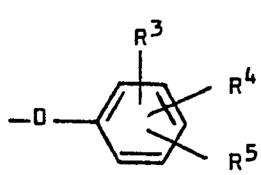

where $R^3$, $R^4$ and $R^5$ are each, independently of one another, hydrogen, halogen, nitro, cyano, carboxyl, alkyl, haloalkyl, alkoxy, haloalkoxy, alkylmercapto, haloalkylmercapto, alkylsulfinyl or alkylsulfonyl, alkyl or alkoxy in each case being of 1 to 4 carbon atoms, and Y is hydrogen, cyano, halomethyl, halogen or nitro.

2. A herbicide containing a pyridazone as claimed in claim 1.

3. A herbicide containing a solid or liquid carrier and a pyridazone as claimed in claim 1.

4. A process for manufacturing a herbicide, wherein a solid or liquid carrier is mixed with a pyridazone as claimed in claim 1.

5. A process for combating unwanted plant growth, wherein the plants or the soil are treated with a pyridazone as claimed in claim 1.

6. A process for the manufacture of a pyridazone as claimed in claim 1, wherein a dihalopyridazone of the formula II

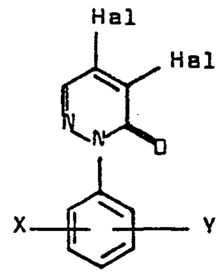

(II)

where X and Y have the above meanings and Hal is halogen, is reacted with not less than twice the stoichiometric amount of an amine of the formula III

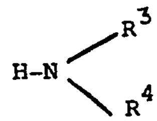

(III)

where $R^3$ or $R^4$ is hydrogen, alkyl of 1 to 3 carbon atoms or alkoxy of 1 to 3 carbon atoms, $R^3$ and $R^4$ being identical or different, or with about the stoichiometric amount, per halogen to be reacted, of an alcoholate of the formula IV

$$MOR^5 \qquad (IV)$$

where M is a cation of a metal, especially sodium or potassium, and $R^5$ is alkyl of 1 to 3 carbon atoms, in the presence of an organic solvent, at from 20 to 150 °C, under atmospheric pressure or superatmospheric pressure, and continuously or batchwise.

7. A pyridazone as claimed in claim 1, wherein $R^1$ is chlorine or methoxy, $R^2$ is chlorine or methoxy, $R^3$ is chlorine or hydrogen, $R^4$ is trifluoromethyl, $R^5$ is hydrogen, and Y is nitro or hydrogen.

8. A herbicide containing a pyridazone as claimed in claim 1, wherein $R^1$ is chlorine or methoxy, $R^2$ is chlorine or methoxy, $R^3$ is chlorine or hydrogen, $R^4$ is trifluoromethyl, $R^5$ is hydrogen, and Y is nitro or hydrogen.

**Claims** (for the Contracting state AT)

1. A herbicide containing a pyridazone of the formula

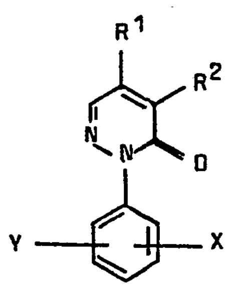

(I)

where $R^2$ is halogen or alkoxy of 1 to 3 carbon atoms, $R^1$ is amino, alkylamino, dialkylamino, alkoxyamino ro alkylalkoxyamino, where alkyl and alkoxy are each of 1 to 3 carbon atoms and the alkyl radicals may be identical or different, halogen, alkoxy of 1 to 3 carbon atoms, trimethyleneimino or amino acylated by $ClH_2CC(O)$- or $CH_3COOCH_2C(O)$-, X is substituted phenoxy of the formula

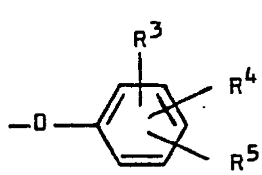

where $R^3$, $R^4$ and $R^5$ are each, independently of one another, hydrogen, halogen, nitro, cyano, carboxyl, alkyl, haloalkyl, alkoxy, haloalkoxy, alkylmercapto, haloalkylmercapto, alkylsulfinyl or alkylsulfonyl, alkyl or alkoxy in each case being of 1 to 4 carbon atoms, and Y is hydrogen, cyano, halomethyl, halogen or nitro.

2. A herbicide containing a solid or liquid carrier and a pyridazone as claimed in claim 1.

3. A process for manufacturing a herbicide, wherein a solid or liquid carrier is mixed with a pyridazone as claimed in claim 1.

4. A process for combating unwanted plant growth, wherein the plants or the soil are treated with a pyridazone as claimed in claim 1.

5. A process for the manufacture of a pyridazone as claimed in claim 1, wherein a dihalopyridazone of the formula II

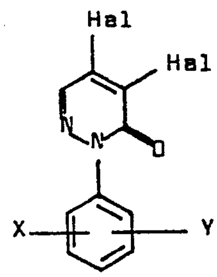

(II)

where X and Y have the above meanings and Hal is halogen, is reacted with not less than twice the stoichiometric amount of an amine of the formula III

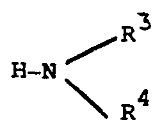

(III)

where $R^3$ or $R^4$ is hydrogen, alkyl of 1 to 3 carbon atoms or alkoxy of 1 to 3 carbon atoms, $R^3$ and $R^4$ being identical or different, or with about the stoichiometric amount, per halogen to be reacted, of an alcoholate of the formula IV

$$MOR^5 \qquad (IV)$$

where M is a cation of a metal, especially sodium or potassium, and $R^5$ is alkyl of 1 to 3 carbon atoms, in the presence of an organic solvent, at from 20 to 150 °C, under atmospheric pressure or superatmospheric pressure, and continuously or batchwise.

6. A herbicide as claimed in claim 1, wherein $R^1$ is chlorine or methoxy, $R^2$ is chlorine or methoxy, $R^3$ is chlorine or hydrogen, $R^4$ is trifluoromethyl, $R^5$ is hydrogen, and Y is nitro or hydrogen.

**Revendications** (pour les Etats contractants : BE, CH, DE, FR, GB, IT, LI, NL, SE)

1. Pyridazones substituées de formule générale

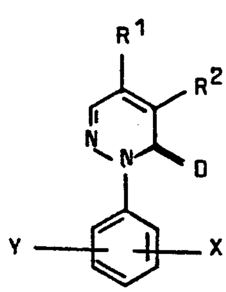

(I)

dans laquelle $R^2$ représente un atome d'halogène ou un reste alcoxy à 1 et 3 atomes de carbone, $R^1$ un groupe amino, alkylamino, dialkylamino, alcoxyamino ou alkyl-alcoxyamino, à 1 à 3 atomes de carbone, chacun un reste alkyle ou alcoxy, les restes alkyle pouvant être identiques ou différents, un atome d'halogène, un reste alcoxy à 1 à 3 atomes de carbone, un reste triméthylénimino ou un groupe acylé par $ClH_2CC(O)$- ou $CH_3COOCH_2C(O)$- X représente un reste phénoxy substitué de formule

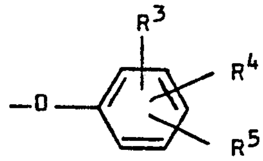

$R^3$, $R^4$ et $R^5$ représentant chacun, indépendamment les uns des autres, hydrogène, halogène, nitro, cyano, carboxyle, alkyle, halogénalkyle, alcoxy, halogénalcoxy, alkylmercapto, halogénalkylmercapto, alkylsulfinyle ou alkylsulfonyle, chacun ayant 1 à 4 atomes de carbone, et Y représente hydrogène, cyano, halogénométhyle, halogène ou nitro.

2. Herbicide, contenant une pyridazone selon la revendication 1.

3. Herbicide, contenant un support solide ou liquide et une pyridazone selon la revendication 1.

4. Procédé de préparation d'un herbicide, caractérisé par le fait qu'on mélange un support solide ou liquide avec une pyridazone selon la revendication 1.

5. Procédé de lutte contre la croissance des plantes indésirables, caractérisé par le fait qu'on traite les plantes ou le sol avec une pyridazone selon la revendication 1.

6. Procédé de préparation d'une pyridazone selon la revendication 1, caractérisé par le fait que, en présence d'un solvant organique, à une température dans la zone comprise entre 20 et 150 °C, sous pression ou sans pression, en continu ou en discontinu, on fait réagir une dihalogénopyridazone de formule II.

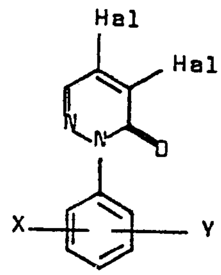

(II)

dans laquelle X et Y ont les significations sus-indiquées et Hal représente un halogène, avec une quantité au moins double d'une amine de formule III

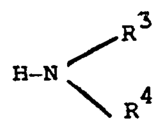

(III)

dans laquelle $R^3$ ou $R^4$ représentent hydrogène, alkyle à 1 à 3 atomes de carbone ou alcoxy à 1 à 3 atomes de carbone et $R^3$ et $R^4$ dans chaque cas sont identiques ou différents, ou avec environ la quantité stoechiométrique par atome d'halogène en réaction d'un alcoolat de formule IV

$$MOR^5 \qquad (IV)$$

dans laquelle M représente un cation métal, en particulier sodium ou potassium, et $R^5$ un reste alkyle à 1 à 3 atomes de carbone.

7. Pyridazone selon la revendication 1, caractérisé par le fait que $R^1$ représente chlore ou méthoxy, $R^2$ chlore ou méthoxy, $R^3$ chlore ou hydrogène, $R^4$ trifluorométhyle, $R^5$ hydrogène et Y nitro ou hydrogène.

8. Herbicide contenant une pyridazone selon la revendication 1, caractérisé par le fait que $R^1$ représente chlore ou méthoxy, $R^2$ chlore ou méthoxy, $R^3$ chlore ou hydrogène, $R^4$ trifluorométhyle, $R^5$ hydrogène et Y nitro ou hydrogène.

**Revendications** (pour l'Etat contractant AT)

1. Herbicide, contenant une pyridazone de formule

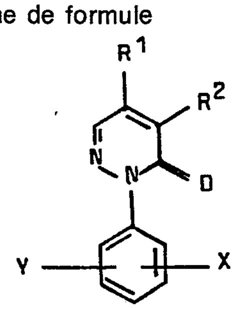

(I)

dans laquelle $R^2$ représente un atome d'halogène ou un reste alcoxy à 1 à 3 atomes de carbone, $R^1$ un groupe amino, alkylamino, dialkylamino, alcoxyamino ou alkylalcoxyamino, à 1 à 3 atomes de carbone, chacun un reste alkyle ou alcoxy, les restes alkyle pouvant être identiques ou différents, un atome d'halogène, un reste alcoxy à 1 à 3 atomes de carbone, un reste triméthylénimino ou un groupe amino acylé par $ClH_2CC(O)$- ou $CH_3COOCH_2C(O)$- X représente un reste phénoxy substitué de formule

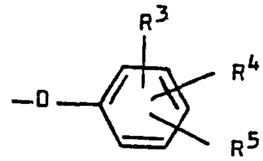

$R^3$, $R^4$ et $R^5$ représentant chacun, indépendamment les uns des autres, hydrogène, halogène, nitro, cyano, carboxyle, alkyle, halogénalkyle, alcoxy, halogénalcoxy, alkylmercapto, halogénalkylmercapto, alkylsulfinyle ou alkylsulfonyle, chacun ayant 1 à 4 atomes de carbone et Y représente hydrogène, cyano, halogénométhyle, halogène ou nitro.

2. Herbicide, contenant un support solide ou liquide et une pyridazone selon la revendication 1.

3. Procédé de préparation d'un herbicide, caractérisé par le fait qu'on mélange un support solide ou liquide avec une pyridazone selon la revendication 1.

4. Procédé de lutte contre la croissance des plantes indésirables, caractérisé par le fait qu'on traite les plantes ou le sol avec une pyridazone selon la revendication 1.

5. Procédé de préparation d'une pyridazone selon la revendication 1, caractérisé par le fait qu'en présence d'un solvant organique, à une température dans la zone comprise entre 20 et 150 °C, sous pression, en continu ou en discontinu, on fait réagir une dihalogénopyridazone de formule II

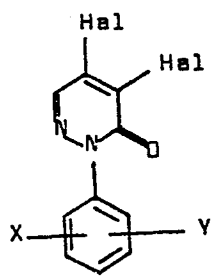

(II)

dans laquelle X et Y ont les significations sus-indiquées et Hal représente un halogène, avec une quantité au moins double d'une amine de formule III

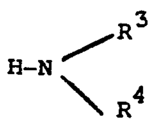

(III)

dans laquelle $R^3$ ou $R^4$ représentent hydrogène, alkyle à 1 à 3 atomes de carbone ou alcoxy à 1 à 3 atomes de carbone et $R^3$ et $R^4$ dans chaque cas sont identiques ou différents, ou avec environ la quantité stoechiométrique par atome d'halogène en réaction d'un alcoolat de formule IV

$$MOR^5 \qquad \text{(IV)}$$

dans laquelle M représente un cation métal, en particulier sodium ou potassium, et $R^5$ un reste alkyle à 1 à 3 atomes de carbone.

6. Herbicide selon la revendication 1, caractérisé par le fait que $R^1$ représente chlore ou méthoxy, $R^2$ chlore ou méthoxy, $R^3$ chlore ou hydrogène, $R^4$ trifluorométhyle, $R^5$ hydrogène et Y nitro ou hydrogène.